(19) Europäisches Patentamt

European Patent Office

Office européen des brevets

(11) **EP 4 606 398 A1**

(12) **EUROPEAN PATENT APPLICATION**

(43) Date of publication:
**27.08.2025 Bulletin 2025/35**

(21) Application number: **24159101.5**

(22) Date of filing: **22.02.2024**

(51) International Patent Classification (IPC):
***A61M 1/00*** *(2006.01)*

(52) Cooperative Patent Classification (CPC):
**A61M 1/96; A61M 1/75; A61M 1/94; A61M 1/964;
A61M 1/966;** A61M 2205/8212

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Mölnlycke Health Care AB
402 52 Göteborg (SE)**

(72) Inventors:
• **BENGTSSON, Erik
431 41 Mölndal (SE)**
• **VANNAS, Alexander
421 51 Göteborg (SE)**

(74) Representative: **Zacco Sweden AB
P.O. Box 5581
Löjtnantsgatan 21
114 85 Stockholm (SE)**

(54) **NEGATIVE PRESSURE WOUND THERAPY**

(57)    An apparatus and related aspects for providing reduced pressure to a tissue site are disclosed. The apparatus comprises a source of negative pressure configured to provide negative pressure via a first fluid flow path from an inlet of the source of negative pressure to a wound site, an electronically controllable valve configured to release negative pressure from the wound site via a second fluid flow path, and a pressure sensor configured to measure a pressure level in the first fluid flow path or the second fluid flow path. The apparatus further comprises control circuitry configured to execute a flushing sequence that is adapted to increase the amount of exudate that is removed from the wound site.

Fig. 2

EP 4 606 398 A1

**Description**

TECHNICAL FIELD

The herein disclosed embodiments generally relate to negative pressure wound therapy (NPWT). In particular, the herein disclosed embodiments relate to apparatuses for providing reduced pressure to a wound site, methods for controlling an apparatus for providing reduced pressure to a wound site, and thereto related computer program products, computer-readable storage media and systems.

BACKGROUND

Negative pressure wound therapy (NPWT) is a technique that promotes healing of e.g. surgical, acute and chronic wounds by the application of a sub-atmospheric pressure ("negative pressure") to the wound, using a negative pressure pump. Wound healing is achieved by applying a negative pressure, such as "vacuum" through a dressing or a cover applied onto the wound. Excess wound exudate is thereby drawn out, which increases the blood flow to the area, and promotes the formation of granulation tissue. The NPWT technique also permits less outside disturbance of the wound and transports excess fluids away from the wound site. NPWT is applicable to a wide variety of wounds such as open wounds, incisional wounds, skin grafts, and the like.

The NPWT technique has, historically, mainly been applied to a patient while in a hospital environment. However, recent product development allows the technique to be used by a patient in a home environment.

In a hospital setting, the wound to be treated is typically an open cavity wound, which is first filled with a wound filler, such as a gauze or a foam. The wound may thereafter be sealed with an adhesive film, and connected to a negative pressure pump via a drain or a port. The size of the foam, gauze and/or the adhesive film may be adapted and cut depending on the size, shape or type of wound. The application procedure is typically carried out by a caregiver. The negative pressure pump used in such a system is typically of a large size and generally has a high capacity to be able to deal with large amounts of wound exudate.

In a home environment, a portable NPWT device, which may be carried around by the patient, is generally preferred. A portable NPWT device typically comprises an absorbent dressing configured to be connected to a negative pressure source by means of a tubing. The pump used is in such devices is typically of a smaller size, and has a more limited capacity. An NPWT system comprising an absorbent dressing is also utilized in a hospital or care facility, particularly on less exuding or "closed" wounds, such as surgically closed incisions.

In some of the portable NPWT systems, the dressing serves as the sole means to collect wound exudate, whereas in other portable NPWT systems and in the NPWT systems adapted for the treatment of wounds in a hospital setting, a fluid collection means, such as a canister, arranged remote from the wound site, is included. In such systems, the canister serves as the predominant means for collection of wound exudate.

Regardless of whether the NPWT system comprises a non-absorbent or an absorbent dressing or whether the application procedure is to be carried out by a caregiver or a wearer in his/her home environment, there is room for improvements in this field. Not only does an exuding wound cause great discomfort and/or pain to the affected person, but it also causes difficulties to nursing personnel and other caregivers. There is therefore a need for new and improved NPWT systems that allow for efficient exudate transport without adding unnecessarily to the complexity and cost of the product.

SUMMARY

The herein disclosed technology seeks to mitigate, alleviate or eliminate one or more of the above-identified deficiencies and disadvantages in the prior art to address various problems relating to transport of exudate from a wound site in an NPWT system.

Various aspects and embodiments of the disclosed technology are defined below and in the accompanying independent and dependent claims.

An aspect of the disclosed technology comprises an apparatus for providing negative pressure to a wound site. The apparatus comprises a source of negative pressure configured to provide negative pressure via a first fluid flow path from an inlet of the source of negative pressure to a wound site, an electronically controllable valve configured to release negative pressure from the wound site via a second fluid flow path, and a pressure sensor configured to measure a pressure level in the first fluid flow path or the second fluid flow path. The apparatus further comprises control circuitry operatively connected to the source of negative pressure, the electronically controllable valve, and the pressure sensor, where the control circuitry is configured to regulate the negative pressure provided to the wound site over a plurality of pressure regulation cycles, each pressure regulation cycle comprising a pressure regulation period and a flushing period. The control circuitry is further configured to, during the pressure regulation period, maintain, at the wound site, a pressure level that is between an upper pressure value and a lower pressure value, by activating and deactivating the negative pressure source. The control circuitry is further configured to, during the flushing period, activate the source of negative pressure until reaching a first pressure value, in response to reaching the first pressure value - open the electronically controllable valve, in response to reaching a second pressure value higher than the first pressure value - close the electronically controllable valve, and activate the source of negative pressure until reaching a target pressure value. The target pressure value is a pressure value within a closed interval defined by the upper pressure value and the lower pressure value.

Another aspect of the disclosed technology comprises a computer-implemented method for operating an apparatus for providing negative pressure to a wound site. The apparatus comprises a source of negative pressure configured to provide negative pressure via a first fluid flow path from an inlet of the source of negative pressure to a wound site, and an electronically controllable valve configured to release negative pressure from the wound site via a second fluid flow path. Moreover, the apparatus is operable to regulate the negative pressure provided to the wound site over a plurality of pressure regulation cycles, each pressure regulation cycle comprising a pressure regulation period and a flushing period. The method comprises, during the pressure regulation period, maintaining, at the wound site, a pressure level that is between an upper pressure value and a lower pressure value, by activating and deactivating the negative pressure source. The method further comprises, during the flushing period, activating the source of negative pressure until reaching a first pressure value, in response to reaching the first pressure value - opening the electronically controllable valve, in response to reaching a second pressure value higher than the first pressure value - closing the electronically controllable valve, and activating the source of negative pressure until reaching a target pressure value. Here, the target pressure value is a pressure value within a closed interval defined by the upper pressure value and the lower pressure value. With this aspect of the disclosed technology, similar advantages and preferred features are present as in the other aspects.

Another aspect of the disclosed technology comprises a computer program product comprising instructions which, when the program is executed by a computing device of an apparatus for providing negative pressure to a wound dressing, causes the apparatus to carry out the method according to any one of the embodiments disclosed herein. With this aspect of the disclosed technology, similar advantages and preferred features are present as in the other aspects.

Another aspect of the disclosed technology comprises a (non-transitory) computer-readable storage medium comprising instructions which, when executed by a computing device of an apparatus for providing negative pressure to a wound dressing, causes the apparatus to carry out the method according to any one of the embodiments disclosed herein. With this aspect of the disclosed technology, similar advantages and preferred features are present as in the other aspects.

The term "non-transitory," as used herein, is intended to describe a computer-readable storage medium (or "memory") excluding propagating electromagnetic signals, but are not intended to otherwise limit the type of physical computer-readable storage device that is encompassed by the phrase computer-readable medium or memory. For instance, the terms "non-transitory computer readable medium" or "tangible memory" are intended to encompass types of storage devices that do not necessarily store information permanently, including for example, random access memory (RAM). Program instructions and data stored on a tangible computer-accessible storage medium in non-transitory form may further be transmitted by transmission media or signals such as electrical, electromagnetic, or digital signals, which may be conveyed via a communication medium such as a network and/or a wireless link. Thus, the term "non-transitory", as used herein, is a limitation of the medium itself (i.e., tangible, not a signal) as opposed to a limitation on data storage persistency (e.g., RAM vs. ROM).

Another aspect of the disclosed technology comprises a wound treatment system comprising apparatus according to any one of the embodiments disclosed herein, a wound cover for creating a sealed space defined in part by the wound site, and a tubing assembly defining the first fluid flow path and the second fluid flow path.

The disclosed aspects and preferred embodiments may be suitably combined with each other in any manner apparent to anyone of ordinary skill in the art, such that one or more features or embodiments disclosed in relation to one aspect may also be considered to be disclosed in relation to another aspect or embodiment of another aspect.

An advantage of some embodiments is that an improved exudate transport from the wound site may be achieved for dual lumen negative pressure wound therapy systems. Thereby the effects of the therapy may be improved as less exudate is present at the wound site. Moreover, the risk of backflow in the exudate lumen is reduced.

An advantage of some embodiments is that the system is more reactive to changes in conditions affecting the wound therapy system, rendering a more stable provision of therapy with capacity to robustly maintain a desired negative pressure level at the wound site over time.

Further embodiments are defined in the dependent claims. It should be emphasized that the term "comprises/comprising" when used in this specification is taken to specify the presence of stated features, integers, steps, or components. It does not preclude the presence or addition of one or more other features, integers, steps, components, or groups thereof.

These and other features and advantages of the disclosed technology will in the following be further clarified with reference to the embodiments described hereinafter.

## BRIEF DESCRIPTION OF THE DRAWINGS

The above aspects, features and advantages of the disclosed technology, will be more fully appreciated by reference to the following illustrative and non-limiting detailed description of example embodiments of the present disclosure, when taken in conjunction with the accompanying drawings, in which:

Fig. 1 is a schematic illustration of a wound treatment system in accordance with some embodiments.

Fig. 2 is a schematic graph of negative pressure over time for multiple pressure regulation cycles in accordance with some embodiments.

Fig. 3 is a schematic graph of negative pressure over time for multiple pressure regulation cycles in accordance with some embodiments.

Fig. 4 is a schematic graph of negative pressure over time for multiple pressure regulation cycles in accordance with some embodiments.

Fig. 5 is a schematic graph of negative pressure over time for multiple pressure regulation cycles in accordance with some embodiments.

Fig. 6 is a schematic flowchart representation of a method for operating an apparatus for providing negative pressure to a wound site in accordance with some embodiments.

## DETAILED DESCRIPTION

The present disclosure will now be described in detail with reference to the accompanying drawings, in which some example embodiments of the disclosed technology are shown. The disclosed technology may, however, be embodied in other forms and should not be construed as limited to the disclosed example embodiments. The disclosed example embodiments are provided to fully convey the scope of the disclosed technology to the skilled person. Like reference characters refer to like elements throughout. Those skilled in the art will appreciate that the steps, services and functions explained herein may be implemented using individual hardware circuitry, using software functioning in conjunction with hardware circuitry such as a programmed microprocessor or general-purpose computer, using one or more Application Specific Integrated Circuits (ASICs), using one or more Field Programmable Gate Arrays (FPGA) and/or using one or more Digital Signal Processors (DSPs).

It will also be appreciated that when the present disclosure is described in terms of a method, it may also be embodied in an apparatus comprising one or more processors, one or more memories coupled to the one or more processors, where computer code is loaded to implement the method. For example, the one or more memories may store one or more computer programs that cause the apparatus to perform the steps, services and functions disclosed herein when executed by the one or more processors in some embodiments.

It is also to be understood that the terminology used herein is for purpose of describing particular embodiments only, and is not intended to be limiting. It should be noted that, as used in the specification and the appended claim, the articles "a", "an", "the", and "said" are intended to mean that there are one or more of the elements unless the context clearly dictates otherwise. Thus, for example, reference to "a unit" or "the unit" may refer to more than one unit in some contexts, and the like. Furthermore, the words "comprising", "including", "containing" do not exclude other elements or steps. It should be emphasized that the term "comprises/comprising" when used in this specification is taken to specify the presence of stated features, integers, steps, or components. It does not preclude the presence or addition of one or more other features, integers, steps, components, or groups thereof. The term "and/or" is to be interpreted as meaning "both" as well and each as an alternative. Similarly, "at least one of A and B" is to be interpreted as only A, only B, or both A and B.

It will also be understood that, although the term first, second, etc. may be used herein to describe various elements or features, these elements should not be limited by these terms. These terms are only used to distinguish one element from another. For example, a first signal could be termed a second signal, and, similarly, a second signal could be termed a first signal, without departing from the scope of the embodiments. The first signal and the second signal are both signals, but they are not the same signal. The term "operatively connected" is in the context of the present disclosure to be understood as that the "operatively connected" entities or units can transmit and/or receive signals to and/or from each other.

As used herein, the term "in response to" may be construed to mean "when or "upon" or "if" depending on the context. Similarly, the phrase "if it is determined' or "when it is determined" or "in an instance of" may be construed to mean "upon determining or "in response to determining" or "upon detecting and identifying occurrence of an event" or "in response to detecting occurrence of an event" depending on the context. Accordingly, the phrase "if X equals Y" may be construed as "when X equals Y", "when it is determined that X equals Y", "in response to X being equal to Y", or "in response to detecting/determining that X equals Y" depending on the context.

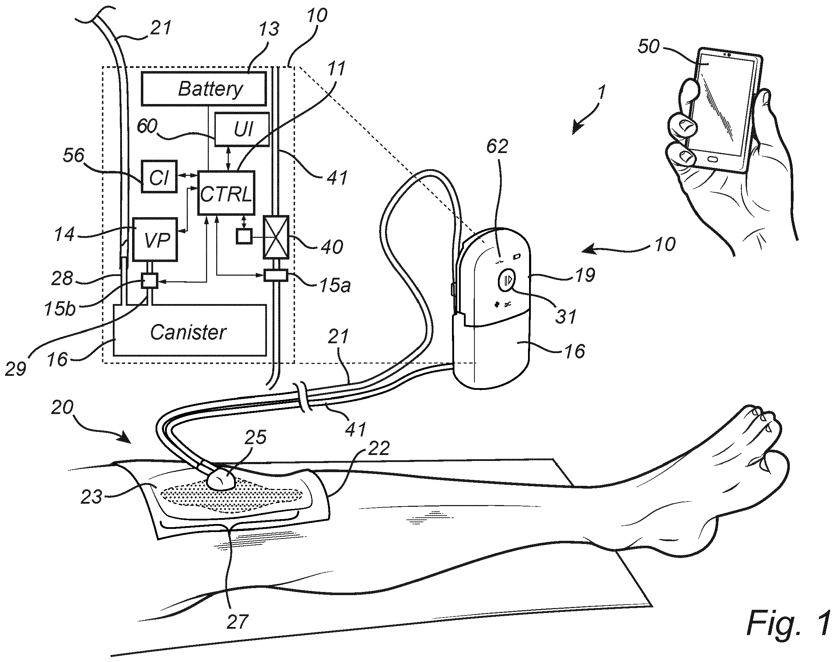

Turning now to the drawings, and to Fig. 1 in particular, there is schematically illustrated a wound treatment system 1 (sometimes simply referred to as "the system" 1) in accordance with some embodiments. The depicted wound treatment system 1 may be referred to as an "NPWT system" 1, "reduced pressure wound treatment system" 1, or "negative pressure wound treatment system" 1. In particular, the depicted wound treatment system 1 may be referred to as a "dual lumen" wound treatment system 1. The wound treatment system 1 comprises an apparatus 10 for providing negative pressure to a wound site 27 (or otherwise referred to as a tissue site 27). The wound treatment system 1 further comprises a wound cover 22 that is adapted to create a sealed space 23 defined in part by a wound surface, such as at the skin of a user/person, at or around a wound of the user/person.

Further, the apparatus 10 (may also be referred to as an NPWT device 10 or pump device 10) is fluidly connected to the wound cover 22 using a tubing assembly having a first tubing 21 at least partly defining a first fluid flow path and a second tubing 41 at least partly defining a second fluid flow path. The tubing 21, 41 may be of any suitable flexible tubing fabricated from elastomeric, polymeric materials, or any other suitable material with similar properties. The tubing 21, 41 may connect to the wound dressing 20, or more specifically to the wound cover 22 of the wound dressing 20, via a suitable connector 25. The connector 25 may be adhered or otherwise attached to the wound cover 22. In particular, the connector 25 may be attached around an opening (not shown) formed in the wound cover 22. The tubing assembly may accordingly comprise two individual tubes.

The expression "wound cover" as used herein should be interpreted broadly as any wound site member, e.g. it can be a film sealed around a periphery of a wound site 27, where a wound filler may be used to fill the wound volume prior to the application of such wound cover. Wound cover may also refer to a backing layer of a wound dressing 20 comprising an additional layer(s) such as for example an absorbent layer and/or a spacer layer.

The apparatus 10 comprises a source of negative pressure 14 configured to provide negative pressure (i.e. sub-atmospheric pressure) via the first fluid flow path from an inlet of the source of negative pressure 14 to the wound dressing 20, the wound site 27, the sealed space 23, or to the wound cover 22. The first fluid flow path ("exudate lumen") is at least partly defined by the first tubing 21. The wound treatment system 1 further comprises a second fluid flow path that fluidly connects the wound site, or the sealed space 23 created by the wound cover 22, to the ambient atmosphere or a "fluid reservoir" (not shown) via an electronically controllable valve 40. The second fluid flow path ("air lumen") is at least partly defined by the second tubing 41. Here, the fluid flow path from the inlet of the source of negative pressure 14 to the wound site 27 may be construed as a first fluid flow path or "exudate lumen", while the fluid flow path from the wound site 27 to the ambient atmosphere or fluid reservoir via the valve 40 may be construed as a second fluid flow path or "air lumen".

The source of negative pressure 14 is indicated as "VP" ("Vacuum Pump") in the figure. In some embodiments, the source of negative pressure 14 comprises a negative pressure pump that together with a motor is adapted for establishing a negative pressure when the source of negative pressure 14 is operating, i.e. when it is in an active state or simply "active". The source of negative pressure may comprise any type of pump and motor that are biocompatible or otherwise suitable for use in an NPWT setting and capable of maintaining or drawing adequate and therapeutic vacuum levels (i.e., negative pressure levels). Preferably, the negative pressure level to be achieved is in a range between about -20 mmHg and about -300 mmHg. In some embodiments, a negative pressure range between about -80 mmHg and about -140 mmHg is used. In some embodiments, a negative pressure range between about -115 mmHg and about -135 mmHg is used. In some embodiments, the negative pressure pump is a diaphragm pump, a peristaltic pump, piezoelectric pump or the like, in which a motor causes the moving parts to draw fluid from the wound site 27.

In the context of the present disclosure, it should be understood that the expressions "negative pressure", "sub-atmospheric pressure", "reduced pressure", or even "vacuum", as used interchangeably herein, generally refer to a pressure less than a local ambient pressure, such as the ambient pressure in a local environment external to a sealed therapeutic environment provided by a wound cover 22 or dressing 20. In many cases, the local ambient pressure may also be the atmospheric pressure at which a patient is located. Thus, the "negative pressure" may be understood as a pressure difference between the ambient pressure and the pressure under the wound cover, where ambient pressure is generally set as 0 mmHg. Unless otherwise indicated, values of pressure stated herein are gauge pressures. Similarly, references to increases in negative pressure typically refer to a decrease in pressure, while decreases in negative pressure typically refer to an increase in pressure.

Further, in some embodiments the apparatus 10 comprises a canister 16 fluidly connected to the negative pressure source 14. The canister 16 may be formed from e.g. moulded plastic or the like, and possibly be a detachable component of the apparatus 10. Moreover, the canister 16 may further be at least partly transparent/translucent to permit viewing into the interior of the canister 16 to assist the user in determining the remaining capacity of the canister 16. As used herein, the term "fluidly connected" should be interpreted broadly and may comprise e.g. any form of tubing, conduits, or channels providing a fluid connection/communication between two components such as between the canister 16 and the negative pressure source 14 or the canister 16 and the wound dressing 20.

In some embodiments, the canister 16 comprises an inlet port 28 for allowing connection to the tubing 21. The inlet port 28 may also be formed elsewhere at the apparatus 10, however still fluidly connected to the canister 16. The connection between the inlet port 28 and the tubing 21 is a sealed connection, thus ensuring that no leakage is formed at the inlet port 28 during normal operation of the apparatus 10. The tubing 21 is preferably detachably connected to the inlet port 28 through conventional means including a friction fit, bayonet coupling, snap fit, barbed connector, or the like. The inlet port 28 may be moulded/formed from the same material and/or at the same time as forming the canister 16. A similar sealed connection (e.g. using a flange insulation/"O-ring") is formed between the canister 16 (at the outlet port 29) and the source of negative pressure 14. The canister 16 may form a part of the first fluid flow path.

In some embodiments, the apparatus comprises a housing 19 enclosing the negative pressure source 14. The canister 16 may be detachably connected to a housing 19 comprising the negative pressure source 14, whereby e.g. a full canister may be removed and replaced with an empty (new) canister. In such embodiments it may be desirable to provide e.g. the canister 16 and the housing 19 with some form of engagement means for securing the canister 16 to the housing such that the canister 16 is not unintentionally removed from the housing 19. The engagement means may in one embodiment comprise a pair of flexible protrusions extending from the canister 16 and adapted to engage with e.g. corresponding locking grooves provided at the housing 19.

However, in some embodiments, the wound treatment system 1 is a canister-less wound treatment system (not shown), where the wound exudate is collected in an absorbent layer, or the like, of the wound dressing 20. In such embodiments, the fluid flow path from the wound dressing 20 to the negative pressure source 14 is provided with one or more suitable filters (not shown) that are adapted to allow gas flow from the wound site 27 to the source of negative pressure 14 and block the flow of liquids from the wound site 27 to the source of negative pressure 14, as readily understood by the person skilled in the art.

In some embodiments, the apparatus 10 comprises a power source, such as e.g. a battery 13 for powering the apparatus 10. The battery 13 may preferably be of the rechargeable type but may alternatively be arranged to be disposable and thus to be changed once discharged. A specifically adapted battery pack may be used in relation to some embodiments. The apparatus 10 may be of single-use type (allowing use during one single treatment time duration) or multiple-use type (allowing use during several different treatment sessions).

Furthermore, the apparatus 10 comprises a pressure sensor 15a arranged in fluid connection with the second fluid flow path and/or a pressure sensor 15b arranged in fluid connection with the first fluid flow path. In some embodiments, the apparatus 10 may alternatively, or additionally, comprise a sensor arranged at or near the wound site 27 (e.g., in the wound dressing 20 or connector 25). Accordingly, the apparatus 10 comprises at least one pressure sensor 15a configured to measure a pressure level in the second fluid flow path, in the first fluid flow path, and/or the wound site. It should however be noted, that regardless of the position of the pressure sensor (e.g., in fluid connection in the first fluid flow path or the second fluid flow path), the pressure as measured by the pressure sensor is considered to be representative of a pressure level at the wound site 27 as it is a closed system. Thus, in some embodiments, the apparatus 10 comprises a pressure sensor arranged to monitor/measure/detect a pressure level at the wound site 27.

In some embodiments, the apparatus 10 comprises a single pressure sensor 15a configured to measure/monitor a pressure level in the second fluid flow path. In the depicted embodiment a pressure sensor 15a is arranged within the housing downstream of the electronically controllable valve 40 (i.e., towards the wound site from the valve 40). However, as readily understood by the skilled person in the art, the one or more pressure sensors 15a may be arranged at any other suitable location to sense or detect a pressure within the second fluid flow path ("air lumen"). In some embodiments, the single pressure sensor is arranged at another suitable location, such as e.g., in connection with the first fluid flow path (see ref. 15b in Fig. 1) or in the wound dressing 20.

In some embodiments, the apparatus 10 comprises one or more pressure sensors arranged in fluid connection with the inlet of the source of negative pressure 14, the canister 16, and/or the sealed space 23. In the depicted embodiment of Fig. 1, the apparatus 10 comprises a pressure sensor 15b arranged in a fluid flow path between the canister 16 and the source of negative pressure 14 (i.e., in fluid connection with the first fluid flow path), in addition to the pressure sensor 15a arranged in fluid connection with the second fluid flow path. However, as readily understood by a person skilled in the art, the additional pressure sensor 15b may be located at any other suitable location to sense or detect a pressure within the fluid flow path between the source of negative pressure 14 and the wound site 27. For example, the additional pressure sensor 15b may be arranged within the canister 16, within the wound dressing 20, or within the connector 25.

The apparatus 10 further comprises control circuitry "CTRL" 11 (may also be referred to as "a control unit", "a controller", or the like) operatively connected to the battery 13, the source of negative pressure 14, the electronically controllable valve 40, the first pressure sensor 15a, and the second pressure sensor 15b. The control circuitry 11 is configured to control operation of the source of negative pressure 14 and the electronically controllable valve 40. The control circuitry 11 may comprise a microprocessor, microcontroller, programmable digital signal processor or another programmable device. The control circuitry 11 may also, or instead, include an application specific integrated circuit (ASIC), a programmable gate array or programmable array logic, a programmable logic device, or a digital signal processor. Where the control circuitry 11 includes a programmable device such as the microprocessor, microcontroller or programmable digital signal processor mentioned above, the control circuitry 11 may further include computer executable code that controls operation of the programmable device. Thus, in some embodiments, the control circuitry 11 comprises one or more processors and a memory, where the memory contains instructions executable by the processor whereby the apparatus 10 is operative to execute any one of the method steps or functions disclosed herein. The term "electronically controllable" should in present context be understood as that one can control the unit or component that is "electronically controllable" with electric signals (control signals), and in particular with electric signals that are output from the control circuitry 11.

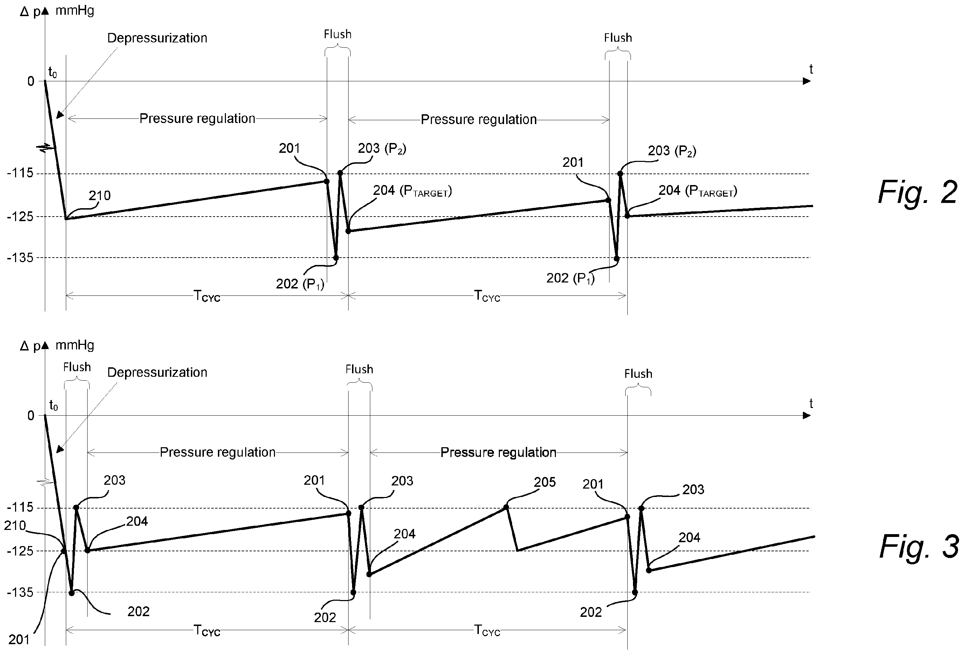

During use of the apparatus 10, the wound cover 22 is arranged at a wound site 27 of the user/patient, forming the sealed space 23. A tubing assembly is provided with a first tubing 21 to fluidly connect the wound cover 22 to the inlet port 28 of the apparatus 10, and a second tubing 41, at least partly defining the second fluid flow path, is closed off from the ambient atmosphere by having the valve 40 closed. The apparatus 10 is then activated, e.g. by a user pressing a start/pause button 31 of a user-interface 60 of the apparatus 10 that activates the source of negative pressure 14. Alternatively, activation may be initiated via an external device 50 connected to the system 1. The external device 50 is further described below. When activated, and the valve 40 is closed, the source of negative pressure 14 will start to evacuate air through the canister 16, the inlet port 28, the tubing 21 and the sealed space 23 formed by the wound cover 22. Accordingly, a negative pressure will be created within the sealed space 23. This initial evacuation of air following a start-up of the apparatus 10 may be referred to as "depressurization" as indicated in Figs. 2-5. The initial evacuation of air (i.e., "depressurization") may continue until reaching a set pressure value (e.g., -125 mmHg). In some embodiments, the control circuitry 11 is configured to control the source of negative pressure 14 so to establish and maintain a negative pressure level (as measured by the pressure sensor(s) 15a, 15b) within a negative pressure range. The negative pressure range may for example be -20 mmHg to -300 mmHg, -80 to -180 mmHg, -100 to -150 mmHg, -110 to - 140 mmHg, -115 to -135 mmHg, or any suitable subrange thereof (e.g., -80 mmHg to -100 mmHg). The negative pressure range may for example be selected in view of the type of wound and/or the patient.

In case a liquid has been formed at the wound site 27, this liquid from the wound site 27 may at least partly be "drawn" from the wound site, through the tubing 21, the inlet port 28 and into the canister 16. The amount of liquid (possibly defined as exudate) that is drawn from the wound and collected in the canister 16 will depend on the type of wound that is being treated, the duration of the treatment, as well as the type of wound dressing 20 used. For example, in case an absorbent dressing is used, the liquid may be absorbed and collected both in the canister 16 and the wound dressing 20, whereas if a dressing with no absorption capacity or little absorption capacity is used, most or all of the liquid from the wound site 27 may be collected in the canister 16. A suitable filter member (not shown) is generally arranged between at the outlet port 29 of the canister 16 to ensure that no liquid is allowed to pass to the source of negative pressure 14 from the canister 16.

The control circuitry 11 is further configured to control an operation of the electronically controllable valve 40 so to release negative pressure from the wound site via the second fluid flow path (or "air lumen") 41. In other words, the control circuitry 11 is configured to open the electronically controllable valve 40 so to introduce fluid (e.g. air) to the wound site 27 when there is a sub-atmospheric pressure under the wound cover 22. This operation may also be referred to as "flushing". Since the wound treatment system 1 depicted in Fig. 1 does not have any "controlled leakage flow", there is a risk that the apparatus 10 would not be able to draw any, or at least a sub-optimal amount of "exudate" from the wound site 27 to the canister 16 once negative pressure has been established under the wound cover 22 due to lack of airflow through the system 1. Therefore, in order to be able to draw desired amounts of wound exudate from the wound site 27, a fluid, such as e.g. air from the ambient atmosphere, is controllably introduced, at defined time intervals and/or in response to pressure measurements, by opening the electronically controllable valve 40. In general, once enough fluid has been introduced (e.g. in response to a negative pressure within the first, second fluid flow path, and/or the wound site 27 reaching a lower negative pressure threshold - i.e., upper pressure threshold), the control circuitry 11 is configured to close the electronically controllable valve 40 and to activate the source of negative pressure 14. However, in some embodiments, the negative pressure source 14 may be active for at least some of the duration that the electronically controllable valve 40 is open.

The ability to controllably "flush" the system 1 by injecting air via an electronically controllable valve 40 may provide the advantage of longer pressure regulation cycles (may also be referred to as NPWT cycles), which reduces the on-time for the source of negative pressure 14 and therefore reduces the energy consumption of the apparatus 10.

The apparatus 10 may further comprise an air filter (not shown) arranged in the second fluid flow path in order to limit the airflow when the electronically controllable valve 40 is open. In some embodiments, the air filter comprises a hydrophobic and porous material, where the size of the pores is configured to allow for a flow in the range of 50-120 ml/s, such as 60-100 ml/s when the valve 40 is opened after negative pressure has been established under the wound cover 20. The pore size of the filter is preferably measured in a non-compressed state. In some embodiments, the air filter comprises polyethylene or sintered polyethylene. The air filter may also serve to reduce the risk of contaminants entering the wound site 27 when the valve 40 is opened (i.e., during "flushing"). The air filter may be arranged in the tube 41 that at least partly defines the second fluid flow path. The air filter may be arranged upstream of the valve 40 (i.e., between the valve 40 and the inlet of ambient air, or downstream of the valve 40 such as between the valve and the pressure sensor 15a.

In some embodiments, during use, the control circuitry 11 is configured to regulate the negative pressure provided to the wound site over a plurality of pressure regulation cycles ($T_{CYC}$). In general, a pressure regulation cycle ($T_{CYC}$) may be understood as a time period comprising at least a "pressure regulation period" and a "flushing period". Stated differently, the apparatus 10 may be operated in a "pressure regulation mode" and a "flushing mode". During the "pressure regulation period" the control circuitry 11 is configured to operate the negative pressure source 14 so to maintain a level of negative pressure in a suitable negative pressure range as exemplified above. In other words, during the "pressure regulation period" the electronically controllable valve 40 is closed and the source of negative pressure 14 is operated to maintain a desired level of negative pressure at the wound site 27.

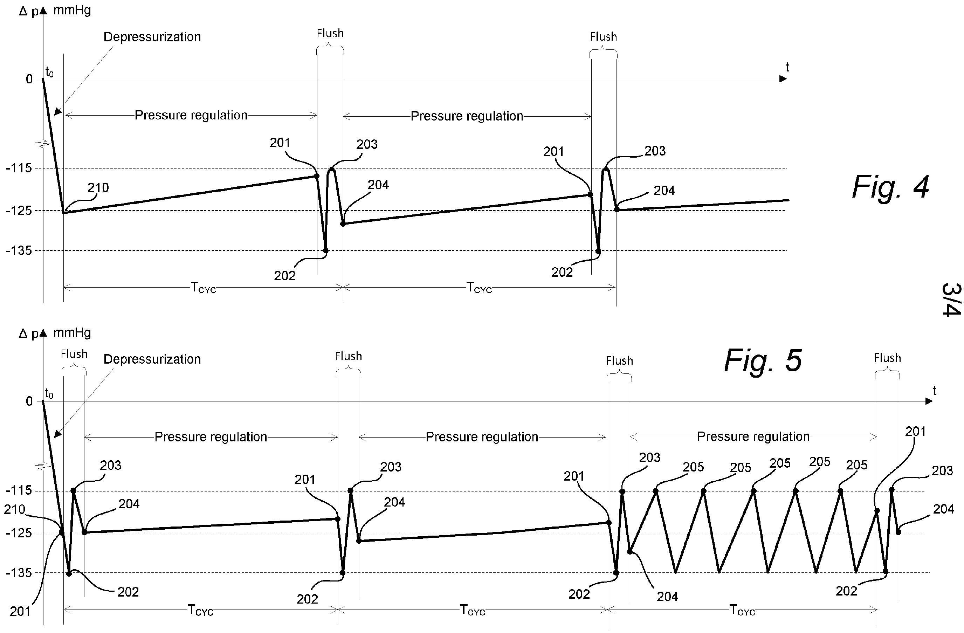

During the "flushing period" the control circuitry 11 is configured to open the electronically controllable valve 40 so to introduce fluid (e.g., air from the ambient atmosphere) to the wound site 27, close the valve 40, and activate the source of negative pressure 14 to draw wound exudate from the wound site and potentially to re-establish an adequate negative pressure level. Accordingly, the flushing period causes a temporary decrease in negative pressure at the wound site 27 (i.e., a temporary increase in pressure). The pressure regulation cycle may comprise a pressure regulation period followed by a flushing period (see e.g., Fig. 2 or Fig. 4) or a flushing period followed by a pressure regulation period (see e.g., Fig. 3 or Fig. 5).

The durations of the pressure regulation periods and flushing periods, and accordingly the duration of a pressure regulation cycle ($T_{CYC}$), may be fixed (i.e., time-controlled) or dynamically controlled based pressure measurements. The duration of a pressure regulation cycle ($T_{CYC}$) may also be dynamically controlled based on a total treatment time such that the pressure regulation cycles ($T_{CYC}$) are shorter during an initial part of the treatment as compared to a later part. Further, in some embodiments, the durations of the pressure regulation periods and flushing periods, and accordingly the duration of a pressure regulation cycle ($T_{CYC}$), may be controlled based on pressure measurements in combination with time-control. For example, during the flushing period the control circuitry 11 may be configured to open the valve until a pressure level (as measured by the sensor(s) 15a, 15b) is reached or for 2 seconds, whichever occurs first. Thus, the time-control aspect may define a longest duration for each period, while detection of certain pressure levels may shorten the duration. Further details related to the pressure regulation cycle, pressure regulation period, and flushing period are provided with reference with Figs. 2-5 below.

Moving on, the apparatus 10 utilizes a flushing algorithm or flushing sequence that is adapted to increase the amount of exudate that is removed from the wound site 27 (e.g., transported to the canister 16) during each "flush" as compared to prior known solutions. In more detail, the flushing sequence starts with a depressurization (i.e., an increase of negative pressure) until a first set pressure value 202 ($P_1$) is reached. Once the first set pressure value 202 ($P_1$) is reached, the pump 14 is shut off and the valve 40 is opened, and kept open until a second set pressure value 203 ($P_2$) is reached, thereby increasing the pressure at the wound site 27 (i.e., decreasing the negative pressure). The pump 14 may however be kept on for an initial portion of the time after the valve 40 is opened.

When the second set pressure value 203 ($P_2$) is reached, the valve 40 is again closed, and the pump 14 is again activated until a target pressure value 204 ($P_{TARGET}$) is reached. Here, one may however apply some overlap between the pump 14 activation and the closing of the valve 40 such that the pump 14 is activated before the valve 40 is closed. The target pressure value 204 ($P_{TARGET}$) is a pressure value that is within the range defined by the upper and lower values used during the pressure regulation period (-115 mmHg and -135 mmHg in the depicted embodiments of Figs. 2-5). In some embodiments, the target pressure value 204 ($P_{TARGET}$) may be set in dependence of an average pressure value of one or more preceding pressure regulation cycles ($T_{CYC}$) or an average pressure value of one or more preceding pressure regulation periods.

By depressurizing before the valve 40 is opened, more air can be introduced into the system and more exudate can therefore be transported away from the wound site 27, while still maintaining the pressure within therapeutic limits during the flushing sequence. Without the initial depressurization one would likely have to allow the pressure to increase to a higher value (i.e., the negative pressure to decrease to a lower value) in order to obtain a similar effect, which may push the pressure at the wound site outside of the desired therapeutic limits (e.g., the limits imposed during pressure regulation). Additionally, by performing the flushing at lower pressures (enabled by the depressurization at the start of the flushing sequence) the air may be introduced at a higher pace into the system, thereby reducing the duration of the flushing sequence. Moreover, if the flushing sequence is to adhere to the pressure limits used during pressure regulation, then if the pressure is close to the upper pressure limit at the start of a flushing sequences, that flushing sequence may be without effect as no significant amount of air was introduced into the system.

Further, by finishing the flushing sequence at a target value 204 ($P_{TARGET}$) that is set in dependence of an average pressure value of one or more preceding pressure regulation cycles or one or more preceding pressure regulation periods, the chances of maintaining a desired negative pressure level over a longer time may be increased since the apparatus 10 is more responsive to temporary conditions that may occur between pressure regulation cycles (e.g., increase/decrease of leakage).

Accordingly, the apparatus 10 comprises control circuitry 11 configured to regulate the negative pressure provided to the wound site 27 over a plurality of pressure regulation cycles ($T_{CYC}$), where each pressure regulation cycle comprises a pressure regulation period and a flushing period.

During the pressure regulation period, the control circuitry 11 is configured to maintain, at the wound site 27, a pressure level that is between an upper pressure value and a lower pressure value, by activating and deactivating the source of negative pressure 14. The upper pressure value and the lower pressure value may for example respectively be -80 mmHg and -140 mmHg, -100 mmHg and -140 mmHg, -115 mmHg and -135 mmHg, -120 mmHg and -135 mmHg, or any other suitable negative pressure range. In the depicted embodiments of Figs. 2-5, the upper pressure value and the lower pressure value are set at -115 mmHg and -135 mmHg, respectively. The terms "pressure level" and "pressure value" are used interchangeably herein, and sometimes mixed in order to increase readability when referring to one or the other.

During the pressure regulation period, the control circuitry 11 may be configured to activate the source of negative pressure 14 in response to the pressure level reaching the upper pressure value 205, and to deactivate the source of negative pressure 14 in response to the pressure level reaching some desired pressure value, such as for example, a value in the middle of the upper and lower pressure values as indicated in Fig. 3. Alternatively, the control circuitry 11 may be configured to activate the source of negative pressure 14 in response to the pressure level reaching the upper pressure value 205, and to deactivate the source of negative pressure 14 in response to the pressure level reaching the lower pressure value, as indicated in Fig. 5.

The pressure regulation period be of any suitable length as required by the applicable scenario, and may for example be specified based on the patient, type of wound, and/or any other relevant circumstances. In some embodiments, the pressure regulation period is of a length in the range of 5 min to 120 min, such as for example, 20 min, 30 min, 40 min, 60 min, or 90 min. As mentioned in the foregoing, the length of the pressure regulation period need not be static but may for example be shorter (e.g., 10 min) during an initial portion of a therapy cycle, and longer (e.g., 40 min) at an end portion of the entire therapy cycle.

In some embodiments, the control circuitry 11 is configured to start/reset a counter/timer once it has completed a flushing period. Then, once the counter or timer reaches a threshold (e.g., zero), the apparatus 10 enters the flushing period (flushing mode), and then resets the counter/timer again upon ending the flushing period. The counter/timer may be reset to different values or triggered at different values in dependence of the total runtime of the apparatus 10 (i.e., the time passed since initial start-up of the apparatus 10). For example, the counter/timer may be set to 10 minutes during the first 24 hours since initial start-up, to 20 minutes during the subsequent 24 hours since initial start-up (i.e., between 24 and 48 hours since start-up) and then to 30 minutes from 48 hours since initial start-up until the entire therapy cycle is completed.

Moving on, during the flushing period, the control circuitry 11 is configured to activate the source of negative pressure 14 (as indicated by ref. 201 in Figs. 2-5) until reaching a first pressure value 202 ($P_1$). During this initial part of the flush sequence, the electronically controllable valve 40 is preferably closed. Then, in response to reaching the first pressure value 202 ($P_1$), the control circuitry 11 is configured to open the electronically controllable valve 40, and deactivate the negative pressure source 14.

Further, in response to reaching a second pressure value 203 ($P_2$), that is higher than the first pressure value 202 ($P_1$), the control circuitry 11 is configured to close the electronically controllable valve 40, and activate the source of negative pressure 14 until reaching a target pressure value 204 ($P_{TARGET}$). Here, the target pressure value 204 ($P_{TARGET}$) is a pressure value within a closed interval defined by the upper pressure value and the lower pressure value (from the pressure regulation period). In other words, the target pressure value ($P_{TARGET}$) is set to a value between the boundary pressure values used during the pressure regulation period (between -115 mmHg and -135 mmHg in the depicted embodiments of Figs. 2-5).

It should be noted that even though the first pressure value 202 ($P_1$) and the second pressure value 203 ($P_2$) are chosen to be the same as the upper and lower pressure values from the pressure regulation period in the depicted embodiments of Figs. 2-5, the first pressure value 202 ($P_1$) and the second pressure value 203 ($P_2$) may be independently set and may differ from the upper and lower pressure values from the pressure regulation period. Thus, in some embodiments, the first pressure value 202 ($P_1$) is lower than the lower pressure value. In some embodiments, the second pressure value 203 ($P_2$) is higher than the upper pressure value.

The term "in response to reaching a pressure value" may be understood as "in response to a pressure level, as derived based on an output from the pressure sensor over time, reaching or surpassing a pressure value", "in response to the pressure sensor detecting a pressure level being equal to the pressure value", or "in response to a pressure level, as measured by the pressure sensor, reaching or surpassing a pressure value". Naturally, the pressure level need not be exactly equal to the pressure value (first and second pressure values) to be trigger the actuation of the pump 14 or valve 40. As readily understood by the skilled reader, it may suffice that the pressure level surpasses the applied threshold, for example if the sample rate of the pressure sensor is not high enough to actually register the exact pressure value at every instant. For example, in reference to the term "in response to reaching the first pressure value 202 ($P_1$)" may be construed as "in response to the pressure sensor detecting a pressure level at or below the first pressure value 202 ($P_1$)". Similarly, the term "in response to reaching a second pressure value 203 ($P_2$)" may be construed as "in response to the pressure sensor detecting a pressure level at or above the second pressure value 203 ($P_2$)".

Moreover, the term "reaching" or "surpassing" in reference to the pressure values should be understood as a trigger is considered to be detected when one can conclude that the applicable threshold or set value has been accomplished. For example, assuming that the first pressure value is -135 mmHg, and the pressure level starts from -120 mmHg when the pump 14 is activated. Then, in some scenario the pressure sensor may detect a pressure level of -134,9 mmHg, and in a subsequent sample -135,1 mmHg. In such a scenario, the measurement/detection of -135,1 mmHg is considered to fulfil the "in response to reaching the first pressure value" trigger.

Moving on, in some embodiments, the target pressure value 204 ($P_{TARGET}$) is set in dependence of a pressure level over time measured by the pressure sensor 15a, 15b during a preceding time period. In other words, the preceding pressure measurements affect what the target pressure value 204 ($P_{TARGET}$) is set to.

In some embodiments, the target pressure value 204 ($P_{TARGET}$) is set in dependence of an average pressure level measured by the pressure sensor 15a, 15b during a preceding time period. The preceding time period may be an arbitrary time period such as e.g., the last 30 minutes, the last 60 minutes, or the like. In some embodiments, the preceding time period comprises one or more preceding pressure regulation periods. However, in some embodiments, the preceding time period comprises one or more preceding pressure regulation cycles ($T_{CYC}$). Thus, the preceding time period may comprise the preceding pressure regulation period(s) (i.e., including pressure regulation periods while excluding the flushing periods), or it may comprise the preceding pressure regulation cycle(s) (i.e., including pressure regulation periods and flushing periods).

However, in some embodiments, the time period extends from an initial pump-down following an initial activation of the apparatus 10 up until the activation of the source of negative pressure 14 until reaching the first pressure value during the flushing period. In reference to Figs. 2-5, this would include the time from reference no. 210 to reference no. 201 for each flushing period. However, in some embodiments this may include the time from the initial start of the apparatus 10 (i.e., from time $t_0$ in Figs. 2-5) up until the activation of the pump at the start of each flushing period (i.e., to ref 201 in Figs. 2-5). However, in the embodiments where the pressure regulation cycle starts with a flushing period (Fig. 3 and Fig. 5), the target pressure value 204 ($P_{TARGET}$) may be set to a value in the middle of the upper pressure value and the lower pressure value for the first flushing period.

A purpose of the pressure regulation, and NPWT in general, is to maintain some desired pressure level at the wound site, denoted as $P_{DES}$ in the following. For NPWT systems with a constant leakage airflow ("single lumen" systems) this is generally achieved by regulating the pressure between two pressure thresholds around the desired pressure level ($P_{DES}$). However, for systems without a constant leakage airflow ("dual lumen" systems) the apparatus 10 regulates far more seldom or not at all, meaning that the actual pressure level at the wound site could be offset from the desired pressure level ($P_{DES}$) for longer periods of time. In order to reduce this offset, it is herein proposed to start each pressure regulation period at a target pressure value ($P_{TARGET}$) that is dependent by the pressure level of one or more previous pressure regulation cycles ($T_{CYC}$). As mentioned in the foregoing, a pressure regulation cycle may be defined as the time interval between the end of a flushing period to the end of a subsequent flushing period.

The target pressure value ($P_{TARGET}$) may be calculated after each pressure regulation cycle using the following formula.

$$P_{TARGET} = P_{DES} \cdot (N + 1) - (P_{AVG} \cdot N)$$

$$Lower\ pressure\ value \leq P_{TARGET} \leq Upper\ pressure\ value$$

Here, $P_{AVG}$ is the average pressure, as measured by the pressure sensor(s), of the preceding pressure regulation cycle(s), N is the number of pressure regulation cycles so far that are being accounted for, $P_{TARGET}$ is as mentioned the target pressure value, and $P_{DES}$ is as mentioned the desired pressure level that the apparatus 10 is configured to maintain during therapy. Moreover, the target pressure value ($P_{TARGET}$) is also bound by boundary conditions defined by the upper and lower pressure values used during the pressure regulation period. In other words, the target pressure value is "capped" by the upper and lower pressure values so to ensure a safe operation of the apparatus. Thus, even if the formula would output a target pressure value ($P_{TARGET}$) outside of the boundaries, the target pressure value ($P_{TARGET}$) is simply set to the closest boundary value. Moreover, the average pressure value ($P_{AVG}$) may be derived by summing the pressure samples detected/obtained during the relevant time period and dividing that sum with the number of samples.

As readily understood by the skilled reader, if one only wants to set the target pressure value ($P_{TARGET}$) in dependence of the average pressure of the (directly) single preceding pressure regulation cycle, the factor N is simply set to 1, and the $P_{AVG}$ is the average pressure of that pressure regulation cycle. Similarly, if one wants to regulate against all preceding pressure regulation cycles since the start of the therapy, N is simply set to the number of pressure regulation cycles that have been executed so far, and $P_{AVG}$ is the average pressure of those pressure regulation cycles. One advantage with only looking back towards 1 or 2 pressure regulation cycles for setting the target pressure value ($P_{TARGET}$) is that the system is more reactive, and can therefore quickly adapt to temporary changes experienced by the system.

However, one advantage with looking back over longer periods of time (i.e., multiple pressure regulation cycles) is that the system is more robust and stable.

Further, in some embodiments, the activation of the source of negative pressure 14, during the flushing mode, until reaching the target pressure value 204 ($P_{TARGET}$), occurs prior to the closing of the electronically controllable valve 40. Thereby a smoother pressure curve may be achievable, reducing the risk of discomfort for the patient do to rapidly changing pressure levels. This is for example indicated in Fig. 4 where the curve around the second pressure value 203 ($P_2$) is flattened. Moreover, it allows for better control of the pressure level as the pressure is changed at a slower rate, thereby reducing the risk of extending outside of allowable pressure thresholds. In some embodiments, a similar procedure is applied around the first pressure value 202 ($P_1$), meaning that source of negative pressure 14 is kept in an active state (turned ON) for some time after the valve 40 is opened in response to reaching the first pressure value 202 ($P_1$) and/or that the valve 40 is opened prior to reaching the first pressure value 202 ($P_1$).

The timing of the activations of the source of negative pressure 14 and the opening of the electronically controllable valve 40 may depend on the configured flow rates of the system. In other words, it may depend on how fast the pump 14 evacuates fluids from the wound site and how fast the air is introduced into the system when the valve is opened. In some embodiments, the valve 40 may be opened to a lesser degree during some or all of the "overlap period" and/or the pump 14 may run on a reduced speed (e.g., by applying a reduced voltage) during some or all of the "overlap period".

Moreover, in some embodiments, the apparatus 10 further comprises a user-interface 60 comprising one or more Light Emitting Diodes (LEDs) 62. The control circuitry 11 may be operatively connected to the user-interface 60 and further configured to, in response to a number of activations of the source of negative pressure 14 during a pressure regulation period exceeding a first number, activate at least one LED of the one or more of the LEDs 62. The first number may be construed as a set threshold, and may for example be 5, 8, or 10. However, the first number may depend on a set length of the pressure regulation period such that it is higher for longer pressure regulation periods and lower for short pressure regulation periods.

By counting the number of activations of the pump 14 during a pressure regulation period one can effectively integrate a leakage detection functionality in the apparatus 10 in a simple manner. In more detail, if the pump is activated "too often" in a dual lumen system, this may be used as an indicator of an unwanted leakage being present in the system 1. The unwanted leakage may for example be due to improperly applied wound cover 22, tears or holes in the tubing or wound cover 22, malfunctioning valve 40, and/or leaky connections between the tubing and other components. An example of the leakage detection functionality is illustrated in Fig 5, where during the third pressure regulation cycle, the pump 14 is being activated (reference no. 205 in Fig. 5) far more often than in the other pressure regulation cycles. By activating one or more LEDs 62, the user or caregiver may be notified of the detected leakage condition and be induced to check the system 1 for any obvious leaks.

Further, in some embodiments, the apparatus 10 comprises communication interface 56 having at least one antenna and at least one transceiver operatively connected to the at least one antenna. The control circuitry 11 may accordingly be operatively connected to the communication interface 56 and further configured to, in response to a number of activations of the source of negative pressure 14 during a pressure regulation period exceeding a first number, transmit a signal to an external device 50.

The communication interface 56 may comprise suitable components (e.g. transceivers, antennas, filters, power amplifiers, etc.) with suitable communication protocols (e.g., Wi-Fi, Bluetooth, Bluetooth Low Energy (BLE), Zigbee, or the like) in order to establish a connection with an external device 50 and to transmit and receive wireless signals to and from the external device 50. Accordingly, the communication interface 56 is configured to transmit and receive wireless signals to and from the external handheld device 50. In more detail, the communication interface 56 comprises one or more transceivers and one or more antennas connected to the one or more transceivers. The one or more transceivers are accordingly configured to transmit signals to an external device 50 via the one or more antennas and to receive signals from an external device 50 via the one or more antennas. For example, the communications interface 56 may include a Wi-Fi transceiver for communicating via a wireless communications network or cellular or mobile phone communications transceivers. In another example, the communication interface 56 may include a short-range wireless transmitter (e.g., a Bluetooth wireless transmitter, etc.) for communicating via a short-range wireless communication transceiver.

The external device may be an external handheld device 50, such as a mobile phone (e.g. a smart phone) or tablet, operatively connected to the apparatus 10. Moreover, the handheld device 50 preferably comprises corresponding hardware and software capabilities to establish a wireless communication link with the apparatus 10 and to transmit and receive signals to and from the apparatus 10. Moreover, the external device may comprise a suitable software application configured to output a Graphical User Interface (GUI) comprising a graphical representation comprising an indication of the leakage in the wound treatment system 1 via a display apparatus of the external device 50.

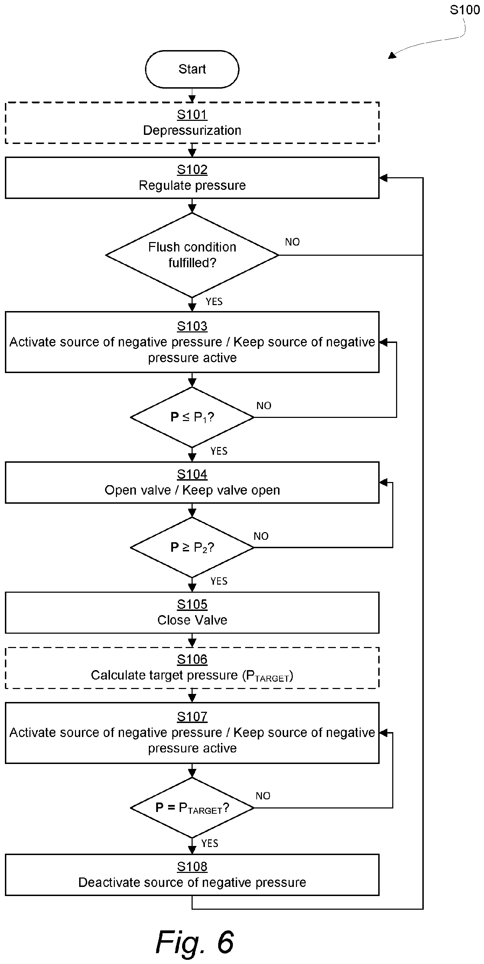

Fig. 6 is a schematic flowchart representation of a computer-implemented method S100 for operating an apparatus for providing negative pressure to a wound site in accordance with some embodiments.

The apparatus may be an apparatus according to any one of the embodiments disclosed herein. The apparatus may accordingly comprise a source of negative pressure configured to provide negative pressure via a first fluid flow path from an inlet of the source of negative pressure to a wound site, and an electronically controllable valve configured to release negative pressure from the wound site via a second fluid flow path. Moreover, the apparatus may be operable to regulate the negative pressure provided to the wound site over a plurality of pressure regulation cycles, each pressure regulation cycle comprising a pressure regulation period and a flushing period. The method S100 is preferably a computer-implemented method S100, performed by a processing system of the apparatus. The processing system may for example comprise one or more processors and one or more memories coupled to the one or more processors, wherein the one or more memories store one or more programs that causes the apparatus to perform the steps, services and functions of any one of the embodiments of the method S100 disclosed herein when executed by the one or more processors.

In some embodiments, the method S100 comprises depressurizing S101 the wound site by activating the source of negative pressure. In other words, the method S100 may comprise turning the source of negative pressure on so to reduce the pressure level at the wound site via the first fluid flow path from an ambient pressure level. The source of negative pressure S101 may be activated S101 until a set pressure value (e.g., -125 mmHg) has been reached (as indicated by one or more pressure sensors). The depressurization S101 is to be construed as a procedure that is generally only carried out upon initial start-up of the apparatus.

Further, the method S100 comprises, during the pressure regulation period, maintaining S102, at the wound site, a pressure level that is between an upper pressure value and a lower pressure value, by activating and deactivating the negative pressure source. The upper pressure value and lower pressure value may for example be predefined or otherwise preconfigured in the apparatus. As mentioned, the upper pressure value may for example be -115 mmHg or -120 mmHg, while the lower pressure value may for example be -135 mmHg or -130 mmHg.

Then, once a flush condition is fulfilled, for example once enough time has gone by since the last flushing period, the apparatus enters a flushing period. Accordingly, the method S100 comprises, during the flushing period, activating S103 the source of negative pressure until reaching a first pressure value, and in response to reaching the first pressure value, opening S104 the electronically controllable valve. Once the first pressure value is reached, the source of negative pressure may be deactivated or shut off. However, as mentioned, there may be some overlap between the valve opening S104 and the running of the source of negative pressure so that the source of negative pressure is deactivated after the opening S104 of the valve. Further, the method S100 comprises, during the flushing period, in response to reaching a second pressure value higher than the first pressure value, closing S105 the electronically controllable valve, and activating S107 the source of negative pressure until reaching a target pressure value, wherein the target pressure value is a pressure value within a closed interval defined by the upper pressure value and the lower pressure value. Moreover, in some embodiments, the method S100 comprises activating S107 the source of negative pressure, until reaching the target pressure value, prior to the closing S105 of the electronically controllable valve.

Further, in some embodiments, the method S100 comprises calculating S106 the target pressure value. The calculation may be done at any suitable point in time during the flushing period, based on e.g., the formula/equation discussed in the foregoing. Thus, in some embodiments, the target pressure value is set in dependence of a pressure level over time (e.g., average pressure level) at the wound site during a preceding time period. The time period may comprise a one or more preceding pressure regulation periods or one or more preceding pressure regulation cycles as discussed in the foregoing.

Furthermore, in some embodiments, the method S100 comprises, in response to a number of activations of the source of negative pressure during a pressure regulation period exceeding a first number, activating at least one LED of one or more of the LEDs comprised by the apparatus. Moreover, in some embodiments, the method S100 comprises, in response to a number of activations of the source of negative pressure 14 during a pressure regulation period exceeding a first number, transmitting a signal, via a communication interface of the apparatus, to an external device 50.

Executable instructions for performing these functions are, optionally, included in a non-transitory computer-readable storage medium or other computer program product configured for execution by one or more processors of an apparatus for providing reduced pressure to a wound dressing.

The herein disclosed technology has been presented above with reference to specific embodiments. However, other embodiments than the above described are possible and within the scope of the claims. Different method steps than those described above, performing the method by hardware or software, may be provided within the scope of the claims. Thus, according to some embodiments, there is provided a non-transitory computer-readable storage medium storing one or more programs configured to be executed by one or more processors of an apparatus of a reduced-pressure wound treatment system, the one or more programs comprising instructions for performing the method S100 according to any one of the above-discussed embodiments. According to some embodiments, there is provided a computer program product comprising instructions which, when the program is executed by a computing device of an apparatus for providing negative pressure to a wound site, causes the apparatus to carry out the method S100 according to any one of above-discussed embodiments.

Generally speaking, a computer-accessible medium may include any tangible or non-transitory storage media or memory media such as electronic, magnetic, or optical media coupled to computer system via bus. The terms "tangible" and "non-transitory," as used herein, are intended to describe a computer-readable storage medium (or "memory") excluding propagating electromagnetic signals, but are not intended to otherwise limit the type of physical computer-readable storage device that is encompassed by the phrase computer-readable medium or memory. For instance, the terms "non-transitory computer-readable medium" or "tangible memory" are intended to encompass types of storage devices that do not necessarily store information permanently, including for example, random access memory (RAM). Program instructions and data stored on a tangible computer-accessible storage medium in non-transitory form may further be transmitted by transmission media or signals such as electrical, electromagnetic, or digital signals, which may be conveyed via a communication medium such as a network and/or a wireless link.

The processor(s) 11 (associated with the apparatus 10) may be or include any number of hardware components for conducting data or signal processing or for executing computer code stored in memory. The device 10 may accordingly have an associated memory, and the memory may be one or more devices for storing data and/or computer code for completing or facilitating the various methods described in the present description. The memory may include volatile memory or non-volatile memory. The memory may include database components, object code components, script components, or any other type of information structure for supporting the various activities of the present description. According to some embodiments, any distributed or local memory device may be utilized with the systems and methods of this description. According to some embodiments embodiment the memory is communicably connected to the processor 11 (e.g., via a circuit or any other wired, wireless, or network connection) and includes computer code for executing one or more processes described herein.

It should be noted that any reference signs do not limit the scope of the claims, that some embodiments may be at least in part implemented by means of both hardware and software, and that several "means" or "units" may be represented by the same item of hardware.

Although the figures may show a specific order of method steps, the order of the steps may differ from what is depicted. In addition, two or more steps may be performed concurrently or with partial concurrence. Such variation will depend on the software and hardware systems chosen and on designer choice. All such variations are within the scope of the appended claims. Likewise, software implementations could be accomplished with standard programming techniques with rule-based logic and other logic to accomplish the generating steps, activating steps, operating steps, causing steps, steps. The above mentioned and described embodiments are only given as examples and should not be limiting to the appended claims. Other solutions, uses, objectives, and functions within the scope of the below described patent claims should be apparent for the person skilled in the art.

**Claims**

1.  An apparatus (10) for providing negative pressure to a wound site, the apparatus (10) comprising:

    a source of negative pressure (14) configured to provide negative pressure via a first fluid flow path from an inlet of the source of negative pressure to a wound site;
    an electronically controllable valve (40) configured to release negative pressure from the wound site via a second fluid flow path;
    a pressure sensor (15a, 15b) configured to measure a pressure level in the first fluid flow path or the second fluid flow path;
    control circuitry (11) operatively connected to the source of negative pressure, the electronically controllable valve, and the pressure sensor, wherein the control circuitry is configured to regulate the negative pressure provided to the wound site over a plurality of pressure regulation cycles ($T_{CYC}$), each pressure regulation cycle comprising a pressure regulation period and a flushing period;
    wherein the control circuitry (11) is further configured to:

        during the pressure regulation period:
        maintain, at the wound site, a pressure level that is between an upper pressure value and a lower pressure value, by activating and deactivating the negative pressure source (14);
        during the flushing period:

            activate the source of negative pressure (14) until reaching a first pressure value (201);
            in response to reaching the first pressure value, open the electronically controllable valve (40);
            in response to reaching a second pressure value (203) higher than the first pressure value (201), close the electronically controllable valve (40);
            activate the source of negative pressure (14) until reaching a target pressure value (204), wherein the target pressure value is a pressure value within a closed interval defined by the upper pressure value and the lower pressure value.

2. The apparatus (10) according to claim 1, wherein the target pressure value (204) is set in dependence of a pressure level over time measured by the pressure sensor (15a, 15b) during a preceding time period.

3. The apparatus (10) according to claim 1, wherein the target pressure value (204) is set in dependence of an average pressure level measured by the pressure sensor during a preceding time period.

4. The apparatus (10) according to claim 2 or 3, wherein the time period comprises a preceding pressure regulation period.

5. The apparatus (10) according to claim 2 or 3, wherein the time period comprises at least one preceding pressure regulation cycle.

6. The apparatus (10) according to claim 2 or 3, wherein the time period extends from an initial pump-down following an initial activation of the apparatus up until the activation of the source of negative pressure (14) until reaching the first pressure value (202) during the flushing period.

7. The apparatus (10) according to any one of claims 1-6, wherein the activation of the source of negative pressure (14), during the flushing mode, until reaching the target pressure value (204) occurs prior to the closing of the electronically controllable valve (40).

8. The apparatus (10) according to any one of claims 1-7, further comprising:
a canister (16) for collecting fluids from the wound site, wherein the canister forms a part of the first fluid flow path between the source of negative pressure (14) and the wound site.

9. The apparatus (10) according to any one of claims 1-8, wherein the apparatus (10) further comprises a user-interface (60) comprising one or more Light Emitting Diodes, LEDs (62);
wherein the control circuitry (11) is operatively connected to the user-interface and further configured to:
in response to a number of activations of the source of negative pressure (14) during a pressure regulation period exceeding a first number:
activate at least one LED of the one or more of the LEDs (62).

10. A computer-implemented method (S100) for operating an apparatus for providing negative pressure to a wound site, wherein the apparatus comprises a source of negative pressure configured to provide negative pressure via a first fluid flow path from an inlet of the source of negative pressure to a wound site, and an electronically controllable valve configured to release negative pressure from the wound site via a second fluid flow path, wherein the apparatus is operable to regulate the negative pressure provided to the wound site over a plurality of pressure regulation cycles ($T_{CYC}$), each pressure regulation cycle comprising a pressure regulation period and a flushing period, the method comprising:

during the pressure regulation period:
maintaining (S102), at the wound site, a pressure level that is between an upper pressure value and a lower pressure value, by activating and deactivating the negative pressure source;
during the flushing period:

activating (S103) the source of negative pressure until reaching a first pressure value (202);
in response to reaching the first pressure value (202), opening (S104) the electronically controllable valve;
in response to reaching a second pressure value (203) higher than the first pressure value, closing (S105) the electronically controllable valve;
activating (S107) the source of negative pressure until reaching a target pressure value (204), wherein the target pressure value is a pressure value within a closed interval defined by the upper pressure value and the lower pressure value.

11. The method (S100) according to claim 10, wherein the target pressure value (204) is set in dependence of a pressure level over time at the wound site during a preceding time period.

12. The method (S100) according to claim 10, wherein the target pressure value (204) is set in dependence of an average pressure level at the wound site during a preceding time period.

13. The method (S100) according to claim 11 or 12, wherein the time period comprises a preceding pressure regulation period.

14. A computer program product comprising instructions which, when the program is executed by a computing device of an apparatus for providing negative pressure to a wound site, causes the apparatus to carry out the method (S100) according to any one of claims 10-13.

15. A system (1) comprising:

an apparatus (10) according to any one of claims 1-9;
a wound cover (22) for creating a sealed space defined in part by the wound site; and
a tubing assembly defining the first fluid flow path and the second fluid flow path.

Fig. 1

EP 4 606 398 A1

Fig. 2

Fig. 3

Fig. 4

Fig. 5

EP 4 606 398 A1

S100

*Fig. 6*

Europäisches
Patentamt

European
Patent Office

Office européen
des brevets

# EUROPEAN SEARCH REPORT

**Application Number**

EP 24 15 9101

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 511 031 B1 (HARTMANN PAUL AG [DE]) 3 May 2023 (2023-05-03) * paragraphs [0134] - [0139]; figures 7,3 * | 1-15 | INV. A61M1/00 |
| X | US 2022/254488 A1 (LOCKE CHRISTOPHER BRIAN [GB] ET AL) 11 August 2022 (2022-08-11) * paragraphs [0059], [0062], [0090], [0092], [0093]; figures 1,4 * | 1-15 | |
| A | US 2013/211318 A1 (CROIZAT PIERRE [DE] ET AL) 15 August 2013 (2013-08-15) * paragraph [0035] * | 1 | |
| A | US 2009/030383 A1 (LARSEN TRUELS STERM [DK] ET AL) 29 January 2009 (2009-01-29) * paragraphs [0104], [0114]; figures 1-3 * | 1 | |

**TECHNICAL FIELDS SEARCHED (IPC)**

A61M

The present search report has been drawn up for all claims

| Place of search | Date of completion of the search | Examiner |
|---|---|---|
| The Hague | 19 March 2024 | Lakkis, Angeliki |

EPO FORM 1503 03.82 (P04C01)

## ANNEX TO THE EUROPEAN SEARCH REPORT
## ON EUROPEAN PATENT APPLICATION NO.

EP 24 15 9101

This annex lists the patent family members relating to the patent documents cited in the above-mentioned European search report.
The members are as contained in the European Patent Office EDP file on
The European Patent Office is in no way liable for these particulars which are merely given for the purpose of information.

19-03-2024

| Patent document cited in search report | | Publication date | Patent family member(s) | | Publication date |
|---|---|---|---|---|---|
| EP 3511031 | B1 | 03-05-2023 | EP | 3187204 A1 | 05-07-2017 |
| | | | EP | 3511031 A1 | 17-07-2019 |
| US 2022254488 | A1 | 11-08-2022 | US | 2022254488 A1 | 11-08-2022 |
| | | | WO | 2020263508 A1 | 30-12-2020 |
| US 2013211318 | A1 | 15-08-2013 | BR | 112014013470 A2 | 13-06-2017 |
| | | | CN | 104039286 A | 10-09-2014 |
| | | | EP | 2626049 A1 | 14-08-2013 |
| | | | EP | 2811955 A1 | 17-12-2014 |
| | | | HK | 1198417 A1 | 24-04-2015 |
| | | | JP | 2015511150 A | 16-04-2015 |
| | | | RU | 2014133706 A | 10-04-2016 |
| | | | US | 2013211318 A1 | 15-08-2013 |
| | | | WO | 2013117318 A1 | 15-08-2013 |
| US 2009030383 | A1 | 29-01-2009 | AU | 2007211738 A1 | 09-08-2007 |
| | | | CA | 2634276 A1 | 09-08-2007 |
| | | | EP | 1986716 A2 | 05-11-2008 |
| | | | JP | 2009525087 A | 09-07-2009 |
| | | | US | 2009030383 A1 | 29-01-2009 |
| | | | WO | 2007087810 A2 | 09-08-2007 |

EPO FORM P0459

For more details about this annex : see Official Journal of the European Patent Office, No. 12/82